# EUROPÄISCHE PATENTSCHRIFT

(11) **EP 3 945 086 B1**
(45) Veröffentlichungstag und Bekanntmachung des Hinweises auf die Patenterteilung: **26.10.2022**
(21) Anmeldenummer: 20188562.1
(22) Anmeldetag: 30.07.2020
(51) Int. Cl.: C07C 51/25, C07C 57/04

(54) **C-4 BASIERTES VERFAHREN ZUR HERSTELLUNG VON MMA UNTER RÜCKFÜHRUNG UND RECYCLIERUNG VON METHACROLEIN**
C-4 BASED METHOD FOR PREPARING MMA WITH RECOVERY AND RECYCLING OF METHACROLEIN
PROCÉDÉ BASÉ SUR LE C-4 DESTINÉ À LA FABRICATION DE MMA À L'AIDE DU RECYCLAGE ET DU RECYCLAGE DE LA MÉTHACROLEINE

(43) Veröffentlichungstag der Anmeldung: 02.02.2022
(73) Patentinhaber: Röhm GmbH, 64295 Darmstadt (DE)
(72) Erfinder: KRILL, Steffen, 64367 Mühltal (DE); KÖLBL, Gerhard, 64579 Gernsheim (DE); DENG, Jalyn, 64295 Darmstadt (DE)
(74) Vertreter: Röhm Patent Association

(56) Entgegenhaltungen:
- EP-A1- 2 158 954
- EP-A2- 0 345 083
- GB-A- 2 004 886

## Beschreibung

### Gebiet der Erfindung

Die vorliegende Erfindung beschreibt ein verbessertes, auf C-4 Rohstoffen basierendes Verfahren für die Produktion von Methacrylaten, insbesondere für die Herstellung von Methacrylsäure und Methylmethacrylat (MMA). Dabei handelt es sich bei den C4-Rohstoffen insbesondere um Isobuten oder tert-Butylalkohol. Des Weiteren werden diverser Ausführungsformen für die effiziente Herstellung dieser Produkte dargestellt.

Die vorliegende Erfindung betrifft insbesondere ein Verfahren zur Herstellung von Methacrylsäure und Methylmethacrylat durch Gasphasenoxidation von Isobuten. Es wird in einem zweiten Gasphasenoxidationsschritt Methacrolein zu Methacrylsäure umgesetzt und dabei nicht umgesetztes Methacrolein von Methacrylsäue abgetrennt. Dieses Methacrolein wird in einer Verdampfungsvorrichtung in Gegenwart verschiedener Gase und Wasserdampf verdampft und gasförmig zusammen mit dem Prozessgas der ersten Oxidationsstufe, in der Methacrolein hergestellt wird, gemischt. Auf diese Weise kann das rezyklierte Methacrolein dem Prozess und der katalytischen Partialoxidation erneut zugeführt werden.

### Stand der Technik

Methylmethacrylat (MMA) wird in großen Mengen zur Herstellung von Polymeren bzw. zusammen mit anderen copolymerisierbaren Verbindungen in Copolymeren eingesetzt. Weiterhin ist Methylmethacrylat ein wichtiger Baustein für diverse, auf Methacrylsäure (MAS) basierender Spezialester, die durch Umesterung mit dem entsprechenden Alkohol hergestellt werden. Hieraus ergibt sich ein großes Interesse an möglichst einfachen, wirtschaftlichen und umweltschonenden Herstellungsverfahren für dieses wichtige chemische Produkt.

Die Herstellung von MMA basiert auf drei möglichen Rohstoffgruppen. Eine davon sind C3- eine andere C4-Bausteine, sowie C2-Bausteine.

Die erste kommerziell wichtige Gruppe stellen C3-Bausteine dar. Hierbei wird MMA überwiegend ausgehend von Blausäure und Aceton über das entstehende Acetoncyanhydrin (ACH) als Zentralintermediat hergestellt. Dieses Verfahren hat den Nachteil, dass sehr große Mengen an Ammoniumsulfat erhalten werden, deren Aufbereitung mit sehr hohen Kosten verbunden ist. Weitere Verfahren, die eine andere Rohstoffbasis als ACH verwenden, sind in der einschlägigen Patentliteratur beschrieben und mittlerweile im Produktionsmaßstab realisiert worden.

Mit wachsender Bedeutung werden heute als C-4 basierte Rohstoffe Isobutylen oder tert-Butanol als Edukte zur Methacrylatherstellung verwendet. Dabei werden diese über mehrere Verfahrensstufen in die gewünschten Methacrylsäurederivate umgewandelt. Als eine dritte Alternative kann auch Methyl-tert-butylether (MTBE), das durch Methanoleliminierung in Isobuten umgewandelt wird, eingesetzt werden.

In dieser C4-basierten Verfahren wird im Allgemeinen Isobutylen oder tert-Butanol zu Methacrolein in einer ersten Stufe oxidiert, welches anschließend zu Methacrylsäure mit Sauerstoff umgesetzt wird. Die erhaltene Methacrylsäure wird nachfolgend mit Methanol in MMA überführt. Nähere Einzelheiten zu diesem Verfahren sind unter anderem in Ullmann's Encyclopedia of Industrial Chemistry 2012, Wiley-VCH Verlag GmbH & Co. KGaA, Weinheim, Methacrylic Acid and Derivatives, DOI: 10.1002/14356007.a16_441.pub2 und in Trends and Future of Monomer-MMA Technologies, SUMITOMO KAGAKU 2004- II dargelegt. Weitere Details allgemein zu MMA und Methacrylsäure Herstellverfahren sowie spezifisch zum mehrstufigen Gasphasenverfahren ausgehend von C4 Bausteinen werden in Krill and Rühling et. al. "Many paths lead to methacrylic acid methyl ester", WILEY-VCH Verlag GmbH & Co. KGaA, Weinheim, doi.org/10.1002/ciuz.201900869 beschrieben.

In der Regel wird die C4-Route vom Steamcrackerprodukt IBEN oder wahlweise auch TBA, welches mittels Gasphasenoxidation im ersten Schritt zu MAL oxidiert wird, aus gestartet. In einer weiteren, zweiten Gasphasenoxidationsstufe wird das intermediär erhaltene Methacrolein (MAL) zu MAS oxidiert. Die gasförmigen Reaktionsprodukte werden in einem nachgeschalteten Quenchschritt abgekühlt und weitestgehend kondensiert. Es ist charakteristisch für das Verfahren, dass die zweite Reaktionsstufe keinen vollständigen Umsatz bezüglich MAL aufweist und nicht umgesetztes MAL in einer Absorptions- und Desorptionseinheit wiedergewonnen wird (Recycle-MAL), um es anschließend als Feed der zweiten Reaktionsstufe wieder zuzuführen.

Isobutylen oder tert-Butanol können mit Luftsauerstoff in der Gasphase am heterogenen Kontakt zu Methacrolein (MAL) und anschließend mittels einer oxidativen Veresterungsreaktion von MAL unter Verwendung von Methanol zu MMA umgesetzt werden. Dieses Verfahren ist unter anderem in US 5,969,178 und US 7,012,039 beschrieben. Auch dieses Verfahren wird in dem Artikel von SUMITOMO (s.o.) beschrieben, wobei ausführlich auf die Nachteile dieses Verfahrens hingewiesen wird, die insbesondere in einem hohen Energiebedarf liegen, was u.a. durch die drucklose Fahrweise bedingt ist. In diesem Verfahren wird die Problematik einer Verdampfung von MAL vermieden, da das Verfahren in der flüssigen Phase stattfindet, somit muss MAL nicht in den gasförmigen Zustand überführt werden und die Problematik der Mischung mit kritischen sauerstoffhaltigen Gasen wird so umgangen. Eine Lösung zur Optimierung des zweistufigen Isobuten Gasphasen Prozesses mit MAS als Zwischenstufe kann hieraus nicht abgeleitet werden.

Problematisch an allen diesen Verfahren ist auch die relativ unbefriedigende Ausbeute, insbesondere bedingt durch hohe Verluste bei den Oxidationsschritten und damit einhergehende CO₂ Bildung. Darüber hinaus muss auf eine weiterhin damit einhergehende Nebenproduktbildung, die aufwendige Verfahrensschritte zur Isolierung des Produkts bedingt, hingewiesen werden. So erreichen alle Verfahren, die von Isobutylen oder equivalenten C-4 basierten Rohstoffen wie TBA oder MTBE ausgehen, in der Gasphasenoxidation an einem heterogenen Katalysatorsystem Selektivitäten von 80% bis 90% pro Verfahrenstufe. Somit wird einige Gesamtausbeute von maximal etwa 65 % bis 70% erreicht. Naturgemäß verläuft das Gasphasenverfahren bei moderatem Drücken zwischen 1 bis 2 bar absolut und erzeugt ein Prozessgas, in dem die Produktkomponente nur zu etwa 4 bis 6 Vol% enthalten ist. Die Isolierung des Wertprodukts vom inertem Gasballast ist entsprechend energetisch aufwändig und verbraucht große Mengen an Kühlenergien sowie Dampf für mehrstufige destillative Aufarbeitungsschritte.

Insgesamt sind somit für die C4-basierte MMA-Herstellung folgenden drei Wege gut bekannt:
Verfahren A, "Tandem C4-Direkt-Oxidationsverfahren", ohne Zwischenisolierung von Methacrolein: Hier wird in einem ersten Schritt Methacrolein aus Isobuten hergestellt, das in einem Verfahrensschritt 2 zu Methacrylsäure oxidiert wird, bevor in einem Verfahrensschritt 3 durch Veresterung von Methacrylsäure mit Methanol MMA erhalten wird. Dieses Verfahren wird in der Literatur auch als "Tandemverfahren" bezeichnet, da das Prozessgas der ersten Stufe ohne Isolierung des Zwischenproduktes Methacrolein direkt zu Methacrylsäure oxidiert wird.

Verfahren B, "Getrennte C4-Direktoxidation": Hier wird, ähnlich wie bei Verfahren A, in einem ersten Verfahrensschritt Methacrolein aus Isobuten hergestellt, das in einem separaten Verfahrensschritt in flüssiger Form isoliert und gereinigt wird, bevor es in einem Verfahrensschritt 3 verdampft und zu Methacrylsäure oxidiert und schließlich in einem Verfahrensschritt 4 durch Veresterung zu MMA umgewandelt wird.

Verfahren C, "Direktes Metha-Verfahren" oder direktes oxidatives Veresterungsverfahren: Auch hier wird in einem ersten Verfahrensschritt Methacrolein in der Gasphase an einem Kontakt I aus Isobuten hergestellt, das ebenfalls zunächst in einem Verfahrensschritt 2 isoliert und zwischengereinigt wird, bevor es in einem Verfahrensschritt 3 direkt oxidativ zu MMA verestert wird. Verfahrensschritt 3 wird in der flüssigen Phase an einem suspendierten Kontakt II durchgeführt. Somit ist die Kombination aus einem Gasphasenschritt und einem Prozesschritt in der Flüssigphase der wesentliche Unterschied dieses Verfahrens im Vergleich zu den Verfahren A und B, bei denen beide Partialoxidationen an unterschiedlichen Kontakten jeweils in der Gasphase durchgeführt werden.

Alle beschriebenen Prozess gemäß Stand der Technik sind gut dokumentiert, einschließlich (i) IHS Chemical Process Economics Program, Review 2015-05, R.J. Chang, Syed Naqvi oder (ii) Vapor Phase Catalytic Oxidation of Isobutene to Methacrylic Acid, Stud. Sci. katal. 1981, 7, 755-767.

Verfahren A und B haben gemeinsam, dass Methacrylsäure das Hauptprodukt ist, das gegebenenfalls in einer Veresterungsreaktion mit Methanol zu Methylmethacrylat umgewandelt werden kann. In der Regel wird für diesen letzten Veresterungsschritt ein aktiver Brönstedt saurer Katalysator verwendet. Es ist möglich, eine gelöste starke Säure in der homogenen Variante zu verwenden. Bevorzugte Säuren sind Schwefelsäure, Methansulfonsäure oder Säuren mit ähnlichem pks Wert. In einer anderen Variante können Ionenaustauscher mit immobilisierten Säurefunktionen eingesetzt werden.

Methode C, das "Direct-Metha-Verfahren", verläuft wie schon beschrieben in den ersten beiden Schritten ähnlich dem Verfahren B, mit Zwischenisolierung von flüssigem Methacolein. Dabei wird aus Methacrolein in der Flüssigphasenoxidation MMA direkt gewonnen und es ist kein Zwischenschritt der Methacrylsäureproduktion notwendig.

Die Verfahren A und B sind daher dadurch gekennzeichnet, dass beim Durchlaufen der zweiten Oxidationsstufe nur eine teilweise Umsetzung des im Gasgemisch enthaltenen MAL stattfindet. Neben Kohlenmonoxid und Kohlendioxid entsteht eine Vielzahl weiterer Reaktionsprodukte, nämlich unter anderem Formaldehyd, Essigsäure, Aceton, Acetaldehyd und Acrolein. In den nachfolgenden Verarbeitungsschritten muss nicht umgesetztes MAL aus dem gewünschten Produkt Methacrylsäure isoliert werden. Üblicherweise wird diese Trennaufgabe durch mindestens einen Destillations- oder Extraktionsschritt durchgeführt. MAL selbst wird aus dem Prozessgas absorbiert und weiterverarbeitet, teilweise gereinigt, durch ein Desorptionsverfahren und gegebenenfalls mindestens einen Destillationsvorgang isoliert. Nach der Absorption und Desorption erfolgt gegebenenfalls eine weitere Destillation. Durch das Vorhandensein leichtflüchtiger, aber kondensierbarer Komponenten zusätzlich zum Acrolein erhält man je nach Trennaufwand Rohmethacrolein mit einigen Bestandteilen an organischen Nebenprodukten, meist ein MAL-Gemisch mit einer MAL-Konzentration > 70 Gew%. Das bedeutet, dass das in flüssiger Form durch Abtrennung von Methacrylsäure erhaltene Rohmethacrolein-Gemisch neben einigen Spurenkomponenten wie Methacrylsäure, Essigsäure, Terephthalsäure und Stabilisatoren auch bestimmte Mengen an Komponenten mit ähnlichen oder niedrigeren Siedepunkten im Vergleich zu MAL enthalten kann. Es gibt verschiedene Verfahren zur Wiedereinführung des flüssigen rezyklierten Methacroleins in das Hauptgassystem, insbesondere vor der zweiten Reaktionsstufe. Der Schritt der Verdampfung des Rohmethacroleins sowie die Vermischung mit anderen Gasen und Dampf, unter anderem die Zugabe sauerstoffhaltiger Gase und insbesondere die Mischung und Rückführung in den Prozess, ist ein komplexer und kritischer Vorgang, der im Stand der Technik nicht befriedigend gelöst ist. Einige dieser sekundären Komponenten haben kritische Flammpunkte und beeinflussen die Explosionseigenschaften der resultierenden Mischungen mit sauerstoffhaltigen Gasen. Der Prozess der Wiedereinführung des rezyklierten Methacroleins in den Hauptprozess, beispielsweise vor der zweiten Reaktionsstufe, stellt einen besonders sicherheitskritischen Schritt im Gesamtprozess dar. Im Stand der Technik sind bereits zahlreiche Vorschläge gemacht worden, um dieses Problem durch unterschiedliche Verfahren zu lösen, um nicht vollständig umgesetztes Methacrolein in den Prozess zurückzuführen. Allerdings ist dieser Stand der Technik nicht befriedigend insbesondere hinsichtlich sicherheitstechnischer Aspekte bei der Rückführung des abgetrennten, nicht umgesetzten Methacroleins nach der zweiten Oxidationsstufe, dessen Konditionierung und Verdampfung sowie Abmischung mit inerten Gasen und sauerstoff-haltigen Gasen und des Weiteren der notwendigen Einstellung mit Wasserdampf um optimale Verhältnisse für den zweiten partiellen Gasphasenoxidations-Schritt zu schaffen.

Bei allen diesen Verfahren gibt es folgende Schwierigkeiten bezüglich sicherheitstechnischer Aspekte, die insbesondere bei Abtrennung und Isolierung der Roh-Methacrolein Fraktion, sowie dessen Verdampfung und Einstellung mit Gasen optimiert werden müssen:
1) Methacrolein wird oft nach dem Abtrennen von der Methacrylsäure in flüssiger Form isoliert. Es muss danach durch Verdampfen in die Gasform gebracht und in ein Gasreaktionssystem eingeführt werden. Das flüssige Recyclingmethacrolein reagiert empfindlich auf die dabei vorliegenden Temperaturen, wobei es oberhalb einer spezifischen Temperatur und innerhalb einer bestimmten Verweilzeit zur Polymerisation, Dimerisierung und sonstigen damit einhergehenden ungewünschten Effekten kommen kann. Daher ist es erforderlich, einen Weg zu finden, bei dem bei gegebener, zur Verdampfung notwendiger Energiezufuhr das flüssige Recyclingmethacrolein in einer Konzentration gehalten wird, bei der die Verweilzeit derart kurz gehalten werden kann, dass eine Polymerisation minimiert oder ganz verhindert werden kann.
2) Recyceltes Methacrolein kann neben einigen Spurenbestandteilen wie Methacrylsäure, Essigsäure, Terephthalsäure und Stabilisatoren bestimmte Mengen an Komponenten mit ähnlichem oder niedrigerem Siedepunkt enthalten. Darüber hinaus stellen Terephthalsäure und oft verwendete Stabilisatoren für den Katalysator der zweiten Stufe ein Katalysatorgift dar, insofern sie zu vermehrter Verkokung und Belegung der Katalysatoren im Langzeitbetrieb führen. Dies wirkt sich wiederum negativ auf die Leistung und Lebensdauer des Katalysators aus. Daher ist notwendig, vor der Wiedereinführung von recyceltem Methacrolein vor der Reaktion in das Gasreaktionssystem der zweiten Stufe die Terephthalsäure und Stabilisatoren, z.B. Hydrochinon (HQ), so weit wie möglich zu entfernen. Wesentliche Probleme, die durch höher als Methacrolein siedende Komponenten entstehen sind weiterhin eine beobachtbare Ablagerung und Belegung vieler Anlagenteile und Rohrleitungen, die im Bereich Verdampfung und Rückführung verwendet werden, insbesondere im Langzeitbetrieb. Die hierdurch entstehenden Probleme führen zu Betriebsstörungen und aufwändigen Stillständen um diese Rückstände zu beseitigen. Auch hierbei besteht erheblicher Verbesserungsbedarf als Aufgabe vorliegender Erfindung.
3) Basierend auf dem Katalysatorzustand haben die beiden temperaturgesteuerten Reaktionszonen unterschiedliche Temperaturniveaus. Der Gasstrom, der die erste Reaktionszone verlässt, muss vor dem Eintritt in die zweite Reaktionszone abgekühlt werden. Dies ist deshalb der Fall, weil bei den nach dem Stand der Technik üblichen Verfahren die Salzbadtemperatur der ersten Stufe, welche als direkter Indikator für die Reaktionstemperatur betrachtet werden kann, um 30°C bis 100°C höher liegt als die Salzbadtemperatur der entsprechenden zweiten Stufe. In der Regel wird das heißere Prozessgas der ersten Stufe durch Mischen mit einem kälteren sauerstoffhaltigen, rezykliertes Methacrolein enthaltenen Gasstrom gekühlt. Durch dieses Vorgehen ist man in der Lage, den Sauerstoffgehalt des Einsatzgases für die zweite Stufe zu erhöhen und gleichzeitig dem Prozess rezykliertes Methacrolein wieder zuzuführen. Dieses Verfahren reagiert in Hinblick auf einen sicheren Betrieb sehr empfindlich auf alle physikalischen Parameter und die Stöchiometrie sowie auf die Verweilzeit und die Qualität der Mischung. Andernfalls würden die höhere Sauerstoffkonzentration und eine bestimmte Verweilzeit innerhalb einer Zone mit einer höheren Temperatur das Risiko eines "Nachverbrennungseffekts" erhöhen. Nachverbrennung ist das Phänomen, das für einige temperaturempfindliche Substrate wie Methacrolein in Form von Gasgemischen bekannt ist. Die entsprechenden Substanzen neigen dabei dazu, in kleinere Fragmente zersetzt zu werden. Dies könnte wiederum zu einem spontanen, sicherheitstechnisch problematischen Druck- und/oder Temperaturanstieg führen.
4) Nicht zuletzt kann Methacrolein in der zweiten Reaktionszone nicht vollständig umgesetzt werden. Bei Umwandlungsraten von 70% bis 85% kann eine vernünftige und gute Selektivität erreicht werden. Bei höheren Umwandlungsraten beginnt die Selektivität jedoch deutlich abzunehmen. Um eine hohe Ausbeute an Methacrylsäure zu erreichen, muss Methacrolein rezykliert werden. Das Recycling besteht aus (i) dem Abschrecken und Quenchen des Prozessgases in thermostatisierter, kondensierter Prozessflüssigkeit und (ii) der Absorption, Desorption und optionalen Destillation des Methacroleins in nachfolgenden apparativen Einheiten. Die darauf folgende Wiedereinführung von Methacrolein umfasst die Verdampfung einer bestimmten Qualität des rezyklierten Methacroleins, und es ist notwendig, das verdampfte Rohmethacrolein mit sauerstoffhaltigen Gasen wie rezykliertem Gas, Luft und optionalem Dampf zu mischen. In einigen Fällen kann sich bei diesem Recyclingschritt aufgrund lokaler Bereiche mit hoher Temperatur oder zu hoher Brennstoffkonzentrationen oberhalb der unteren Explosionsgrenze ein explosives Gasgemisch bilden. Besonders bei längerem operativen, kontinuierlichem Betrieb, in dem einige Anlagenteile und Rohrleitungen Ausfällungen, Verschmutzungen und Ablagerungen von Nebenprodukten und polymeren Materialien aufweisen, kann eine Fehlverteilung beobachtet werden, die in Verbindung mit zu hohen Sauerstoffkonzentrationen zu kritischen Sicherheitsbedingungen führt. Letztendlich führt dies auch zu einer Überdruckentlastung über Sicherheitsentlüftungen oder im schlimmsten Fall sogar zu einer lokalen Explosion beziehungsweise je nach Schweregrad der Störung zu einer Verpuffung.

Eine neue Methode zur Erhöhung der Prozesssicherheit im industriellen Maßstab unter Optimierung der Ausbeute muss daher alle dieser vier diskutierten Aspekte berücksichtigen.

So wird zum Beispiel in USA 7,799,946 und GB 2 004 886 das rezyklierte Methacrolein zunächst durch eine Abschreckkolonne (Quenchen mit Prozessmedium) geleitet, in der die schweren Bestandteile, einschließlich Methacrylsäure kondensiert werden. Das Methacrolein enthaltende Gas wird in einer nachfolgenden Kolonne absorbiert und schließlich desorbiert und von der absorbierten Flüssigkeit gestrippt, um ein Methacrolein enthaltendes Gasgemisch zu erhalten, das in den zweiten Reaktor geleitet werden kann. Hier ist dieses Gasgemisch ein nicht-explosives Gas mit einer ausreichend niedrigen Sauerstoffkonzentration. Dennoch reicht auch bei diesem Ansatz der Sauerstoff nicht aus, um die Reaktion der zweiten Stufe ausreichend und optimal zu versorgen. Hier muss ein zusätzliches sauerstoffhaltiges Gas vor der Reaktion in der zweiten Stufe wieder zugeführt werden.

In EP 3 007 69 wird eine spezielle Sparger-Konstruktion am Einleitungspunkt eines Methacrolein enthaltenden Recyclinggasstroms und dessen Vermischung mit dem Reaktionsgas der ersten Stufe beschrieben. Dies erfolgt, um oben beschriebene Nachverbrennungseffekte, die zur Methacrolein Zersetzung führen, zu verhindern. EP 3 007 69 beschreibt weiterhin eine Methode zur Mischung des heißeren Prozessgases der ersten Stufe mit dem kälteren, Methacrolein enthaltenden Recyclinggasstrom. Dies geschieht durch Sprühinjektion eines Gasstroms, wahlweise gemischt mit Luft, Inertgas und rezirkuliertem Methacrolein durch eine spezielle Anordnung von Zerstäubern bzw. Düsen. Durch diese Injektion wird das heißere Gas, welches z.B. eine Temperatur zwischen 350°C und 400°C aufweist, auf eine resultierende Temperatur zwischen 200°C und 240°C abgekühlt. Bei diesem Ansatz würde das Methacrolein-haltige Gasgemisch bei einer relativ hohen Methacrolein-Konzentration eine sauerstoffreiche Konzentration von bis zu 13 Mol% enthalten. Als Folge davon bildet sich ein explosives Gas. Selbst wenn das Gasgemisch durch die Sprüheinspritzung ausreichend unter die Zündtemperatur abgekühlt würde, können lokale Hot Spots mit der Temperatur oberhalb des Zündpunktes nicht ausgeschlossen werden. Hot Spots werden im Sparger durch teerartige Ablagerungen und problematische polymerisierte Rückstände gebildet und beobachtet. Eine kritische Zone für lokale Ablagerungen solcher Rückstände kann zur Verschmutzung und Verstopfung bestimmter Teile der Rohre und des Spargerbereichs führen. Die beschriebene apparative Umsetzung dieses Konzepts ist zwar prinzipiell geeignet, ist aber insbesondere im kontinuierlichen Dauerbetrieb anfällig für Verschmutzungen, was eine regelmäßige Abstellung notwendig macht, um diese Verunreinigungen, teils teerartig, teils pulverförmig zu entfernen. Die Verunreinigungen können pyrolytisch durch Ausbrennen, durch manuelle Entfernung oder durch Spülen mit geeigneten Medien entfernt werden. Vollständige Säuberung des Apparates ist oft nur nach Ausbau der Spargermimik zu erreichen, was einem mehrtägigen Anlagenstillstand erfordert. Die Verschmutzungen in Rohrleitungen und insbesondere an den Auslassdüsen vor und innerhalb des Spargerbereiches führen zur Fehlverteilung des Methacrolein-haltigen Auslassgases und erzeugen lokale Gasgemische, die zündfähig sind, insofern ist der Betrieb insbesondere nach mehreren Wochen Betrieb nicht als sicher zu betrachten.

In US 3,876,693 und US 3,147,084 wird jeweils das erste Reaktionsgas durch einen Reaktor, der mit einem zusätzlichen Wärmetauscher ausgestattet ist, abgekühlt. Dies erfolgt, um die Temperatur des Reaktionsgases des ersten Reaktors weiter abzusenken. Das Thema der sicheren Wiedereinführung von rezyklierten Methacrolein und Sauerstoff wird nicht behandelt. Aber auch hier könnten die Nebenprodukte, insbesondere TPA, in den Wärmetauscherrohren resublimieren und diese zumindest teilweise verstopfen. Die Wärmeaustauscheffizienz und die Reaktionslaufzeit würden so verringert. Darüber hinaus wäre die Reaktorarchitektur recht komplex und die Herstellungs- und Wartungskosten einer solchen apparativen Lösung sind dementsprechend hoch.

In einem weiteren Vorschlag EP 2 158 954 wird eine spezielle Methode zur Recyclierung des nicht umgesetzten Methacroleins beschrieben. Es wird ein spezieller Apparat zur Verdampfung des Methacroleins beschrieben, bei dem ein flüssiger Methacrolein haltiger Strom auf eine Kolonne mit Packungen aufgegeben und der resultierende MAL haltige Gasstrom direkt dem Oxidationsreaktor zugeführt wird. Höhersiedende Komponenten werden im Sumpf ausgeschleust, sodass Verunreinigungen in diesem Apparat weitgehend vermieden werden. Die Problematik der Gaseinmischung, insbesondere mit sauerstoffhaltigen Gasen, um den Sauerstoffgehalt vor dem zweiten Partialoxidationsreaktor einzustellen und insbesondere die Problematik in einen höheren Druckbereich der Reaktoren zurückzugehen wird nicht befriedigend gelöst. Die Sumpftemperatur des Apparates kann zu weiteren Problemen im Dauerbetrieb führen, weil im Medium Terephthalsäure und polymere Ablagerungen entstehen können. Durch die Kreislauffahrweise des Sumpfes wird ein nicht unerheblicher Teil der schwersiedenden Komponenten in den Aufarbeitungs-Prozess zurückgefahren, was mit einem höheren Energieaufwand verbunden ist. Zusammensetzung des resultierenden MAL-haltigen Gastromes wird im Wesentlichen über Druck und Sumpftemperatur der Mimik und des Apparates bestimmt.

Zusammenfassend lässt sich sagen, dass diese Verfahren des Standes der Technik in Hinblick auf eine sicherheitstechnisch effiziente Verfahrensdurchführung eine erhebliche technische Herausforderung darstellen. Bei der Verdampfung des sogenannten Recycling-Methacroleins, d.h. Methacrolein, das in der zweiten Oxidationsstufe nicht umgesetzt wird, und seiner Injektion zwischen den beiden Oxidationsreaktoren arbeitet man im sogenannten "mageren" Bereich. Dieser magere Bereich liegt sehr nahe an der Explosionsgrenze des Gemisches. Der magere Bereich des Gemisches definiert sich durch einen relativ geringe Konzentration flüchtiger Verbindungen im Gasgemisch, was insbesondere auch Methacrolein einschließt. Grundsätzlich wird bei vorliegender Stoffzusammensetzung unterhalb von 2,5 Vol%, bevorzugt unterhalb von 2,2 Vol% bezüglich der Summe der organischen Verbindungen gearbeitet. Prinzipiell wird das Gasgemisch unkritischer, je geringer der Anteil der organischen Bestandteile ist, je niedriger der Sauerstoffgehalt im Gas ist und je geringer die Verweilzeit und thermische Belastung des Gasgemisches ist. Unkritischer meint hier Ein Vorgehen, bei dem die beschriebenen Probleme der Zersetzung im Sinne einer Nachverbrennung vermieden werden können.

Wird dieses kritische Gemisch mit dem Prozessgas der ersten Reaktorstufe eingespeist, z.B. bei Temperaturen meist über 300°C, kommt es oft zu teerartigen Ablagerungen und problematischen Zusammensetzungen des Speisegases vordem zweiten Oxidationsreaktor. Diese Problematik wird in einer Vielzahl von Dokumenten zum Stand der Technik mit entsprechenden Lösungsvorschlägen beschrieben. Allen diesen Lösungen ist jedoch gemein, dass sie nur durch aufwendige und teure Maßnahmen realisiert werden können. Zudem können viele der Probleme insbesondere im Dauerbetrieb nicht ausreichend kompensiert werden.

An dieser Stelle kann somit festgestellt werden, dass bisher kein auf C4-Rohstoff basierendes Verfahren beschrieben wurde, bei dem der kritische Schritt der Abtrennung und Umsetzung von nicht umgesetztem Methacrolein im Prozessgas ausreichend sicher für einen Langzeitbetrieb beschrieben wurde. Daher besteht hier also ein erheblicher Verbesserungsbedarf.

### Aufgaben

Aufgabe der vorliegenden Erfindung war die Optimierung von C4-basierten Verfahren zur Herstellung von Methacrylsäure bzw. MMA, insbesondere solche Verfahren mit Methacrylsäure als Zwischen- oder Endprodukt. Dabei sollen aus dem Prozess hohe Ausbeuten bei gleichzeitig langer Lebensdauer der eingesetzten Katalysatoren, insbesondere in der zweiten Oxidationsstufe erhalten werden. Darüber hinaus soll das optimierte Verfahren bei unkritischen Sauerstoff- und Volatilkonzentrationen, bzw. bei einer unkritischen Temperatur durchgeführt werden können.

Eine weitere Aufgabe bestand darin, das Verfahren derart zu optimieren, dass dieses mit längeren Prozesszeiten zwischen einzelnen Umrüst- bzw. Reinigungsstillständen betrieben werden kann. Dies betrifft insbesondere den Austausch von Geräteteilen in der so genannten Sparger Sektion zwischen den beiden Oxidationsstufen. Es handelt sich dabei um den Anlagenbereich, in dem MAL, enthaltend diverse Nebenprodukte und Restedukte zusammen mit Sauerstoff, Wasserdampf und Stickstoff gasförmig für die Reaktion in der zweiten Oxidationsstufe zur Verfügung gestellt wird.

Zusammengefasst war es damit insbesondere Aufgabe der vorliegenden Erfindung ein verbessertes C4-basiertes Verfahren zur Herstellung von Methacrylsäure als End- oder Zwischenprodukt, welches mit weniger wartungsbedingten Betriebsunterbrechungen, einer insgesamt höheren Produktivität und Effektivität, sowie insgesamt verbesserten Betriebssicherheit betrieben werden kann, zur Verfügung zu stellen. Ein solches Verfahren sollte insbesondere effektiv die Ablagerung von Verschmutzungen im Bereich der Verdampfung des Recycle -MALs sowie der nachfolgenden Sparger Sektion vermeiden.

Insbesondere betreffen diese Aufgaben dabei ein solches Verfahren, welches auf der zweistufigen katalystischen Gasphasenoxidation von Isobuten beruht. Dazu soll in allen Prozessschritten auf möglichst einfache Weise die Bildung von kritischen Gasgemischen bei kritischen Reaktionsbedingungen vermieden werden. Dies betrifft insbesondere auch das Vermeiden der Bildung von Polymeren und hochsiedenden Komponenten in der Sparger Sektion. Solche Ablagerungen würden sonst zu erhöhten Risiken im Betrieb bzw. zu vermehrten wartungs- und reinigungsbedingten Betriebsunterbrechungen führen.

Weitere nicht explizit genannte Aufgaben können sich aus der Beschreibung oder den Beispielen, sowie aus dem Gesamtzusammenhang der Anmeldung, insbesondere in Hinblick auf den Stand der Technik ergeben.

### Lösung

Die gestellten Aufgaben wurden durch die Bereitstellung eines neuartigen Verfahrens zur Herstellung von Methacrylsäure und / oder Methacrylsäureestern gelöst. Dieses neuartige Verfahren umfasst die folgenden Verfahrensschritte:
(a) Umsetzung von Isobuten, tert-Butanol oder einer Mischung enthaltend Isobuten und/oder tert-Butanol in einem ersten katalytischen Gasphasenoxidationsschritt zu einem hauptsächlich Methacrolein enthaltenden Reaktionsprodukt
(b) Umsetzung des hauptsächlich Methacrolein enthaltenden Reaktionsprodukts aus Verfahrensschritt (a) in einem zweiten katalytischen Gasphasenoxidationsschritt zu einem überwiegend aus Methacrylsäure und nicht umgesetztem Methacrolein bestehenden gasförmigen Reaktionsprodukt
(c) Abtrennung, Vorreinigung und Kondensation eines Teils des in Verfahrensschritt (b) im gasförmigen Reaktionsprodukt anfallenden nicht umgesetzten Methacroleins zu einer flüssigen Recycle-Methacrolein-Fraktion
(d) Überführung der Recycle-Methacrolein-Fraktion in einen gasförmigen Strom
und
(e) Umsetzung dieses gasförmigen Recycle-Methacroleinstroms in einem katalytischen Oxidationsschritt

Das erfindungsgemäße Verfahren ist dabei insbesondere dadurch gekennzeichnet, dass Verfahrensschritt (d) die folgenden Verfahrensaspekte aufweist:
i) gleichmäßige Verteilung der in Schritt c) erhaltenen flüssigen Recycle-Methacrolein-Fraktion in Form von Tröpfchen auf der Oberfläche einer Packung zum Erhalt eines Flüssigkeitsfilms
ii) Inkontaktbringen des in Schritt (i) erhaltenen Flüssigkeitsfilms mit einem 3 bis 21 Vol% Sauerstoff enthaltenden Gasstrom, der eine Temperatur aufweist, die 10 bis 150 °C höher ist als die Temperatur des flüssigen Methacroleins, unter Erhalt einer Recycle-Methacrolein enthaltenden Gasmischung
iii) optional Vermischung des gemäß (ii) erhaltenen Gasstroms mit zusätzlichem Wasserdampf
und
iv) Mischen des resultierende Gasstroms, enthaltend Methacrolein, Sauerstoff, Wasserdampf, Stickstoff, sowie andere organische Verbindungen mit 1 bis 6 Kohlenstoffatomen mit dem Reaktionsprodukt der ersten Oxidationstufe (a)

Bevorzugt handelt es sich bei den in den Schritten (a) und (b) beschriebenen Oxidationsschritten um eine in Gegenwart einer sauerstoffhaltigen Gasmischung und Wasserdampf durchgeführte katalytische Gasphasenoxidation. Besonders bevorzugt wird dies.in einem Rohrreaktor über einem heterogenen Kontakt durchgeführt.

Für Schritt (c) ist es besonders bevorzugt, wenn hier mindestens 80% der gebildeten Methacrylsäure vom nicht umgesetzten Methacrolein abgetrennt werden. Insbesondere bevorzugt werden die Abtrennung und Vorreinigung gemäß Schritt (c) durch folgende Aspekte (i) bis (iii) realisiert:
(i) Quenchen der Reaktionsmischung unter Erhalt eines Prozessgases und einer Prozessflüssigkeit,
(ii) Abtrennen mindestens eines Teils der Nebenprodukte aus der Prozessflüssigkeit durch Kristallisation und
(iii) sequentielle Durchführung von Absorptions- und Desorptionsschritten unter Erhalt einer flüssigen Recycle-Methacrolein-Fraktion und einer Stickstoff, Sauerstoff und Wasserdampf enthaltenden Recycle-Gas-Phase.

Dabei können erfindungsgemäß, wenn auch weniger bevorzugt keine oder nur einzelne dieser Aspekte realisiert werden.

Die in Schritt (c) erhaltene flüssige Recycle-Methacrolein-Fraktion enthält optimalerweise 65 bis 99 Gew% Methacrolein, 0 bis 5 Gew% Methacrylsäure, 0 bis 5 Gew% Wasser, bis 5000 ppm Stabilisatoren, Terephthalsäure, nicht flüchtige Verbindungen, 0,5 bis 10 Gew% Aceton und in Summe 0 bis 5 Gew% Essigsäure, Acetaldehyd und Acrolein. Diese flüssige Fraktion, hauptsächlich enthaltend Methacrolein und insbesondere schwerer flüchtige Anteile, wie Essigsäure und Methacrylsäure, enthält in einer bevorzugten Ausführungsform in Spurenmengen auch Stabilisatoren, wie Hydrochinon oder Derivate hiervon, bzw. N-Oxide wie Tempol oder andere im Stand der Technik beschriebene Inhibitoren sowie deren Mischungen. Somit kann, um Ablagerungen zu verhindern, die Verdampfungssektion mit Inhibitoren versorgt werden. Auf der anderen Seite sind im Bereich von Verdampfung und den Rohleitungen zum Sparger aber bevorzugt geeignete Drainagepunkte installiert, um das Equipment kontinuierlich oder diskontinuierlich in vorgegebenen Intervallen zu reinigen und flüssige, feste oder suspensionsartig vorliegende Rückstände zu entfernen. Somit kann das Equipment im laufenden Betrieb rückstandsfrei gehalten werden bzw. Ablagerungen weitestgehend vermieden werden.

Für Verfahrensschritt (d) ist es wiederum bevorzugt, wenn die Recycle-Methacrolein-Fraktion mittels ein oder mehreren Düsen in Gegenwart von ein oder mehreren Gasströmen mit unterschiedlichen Sauerstoffgehalten versprüht und verteilt wird. Dabei entstehen insbesondere bevorzugt Tröpfchen, die einen durchschnittlichen Durchmesser zwischen 100 und 1000 µm aufweisen. Weiterhin wird bevorzugt ein erster Gasstrom gebildet, der einen Sauerstoffgehalt kleiner 10 Vol% und optional ein zweiter Gasstrom, der einen Sauerstoffgehalt größer 10 Vol% aufweist, gebildet. Das Equipment wird an dieser Stelle bei atmosphärischem oder geringen Überdrücken von bis zu 2 bar betrieben. Temperatur und Drücke sind hierbei so zu wählen, dass der Taupunkt von Methacrolein, sowie Wasser im resultierenden Gasgemisch nicht unterschritten wird. So kondensieren lediglich geringe Mengen der höher flüchtigen Bestandteile mit bestimmten Mengen an Wasser und Essigsäure, was wiederum über wie zuvor beschriebene DrainagePunkte abgeführt werden kann.

Für Schritt (e) gibt es auch diverse bevorzugte Ausführungsformen. So können beispielsweise bevorzugt in Schritt (e)(i) mindestens 50 % der Recycle-Methacrolein-Tröpfchen, mit Tröpfchengrößen kleiner als 500 µm, auf der Packung verteilt werden.

In Schritt (e)(ii) wird wiederum bevorzugt der 3 bis 21 Vol% Sauerstoff enthaltende Gasstrom durch Vermischung von in Schritt (a) und / oder Schritt (b) erhaltenem rückgeführtem Prozessgas und zusätzlicher Luft erhalten.

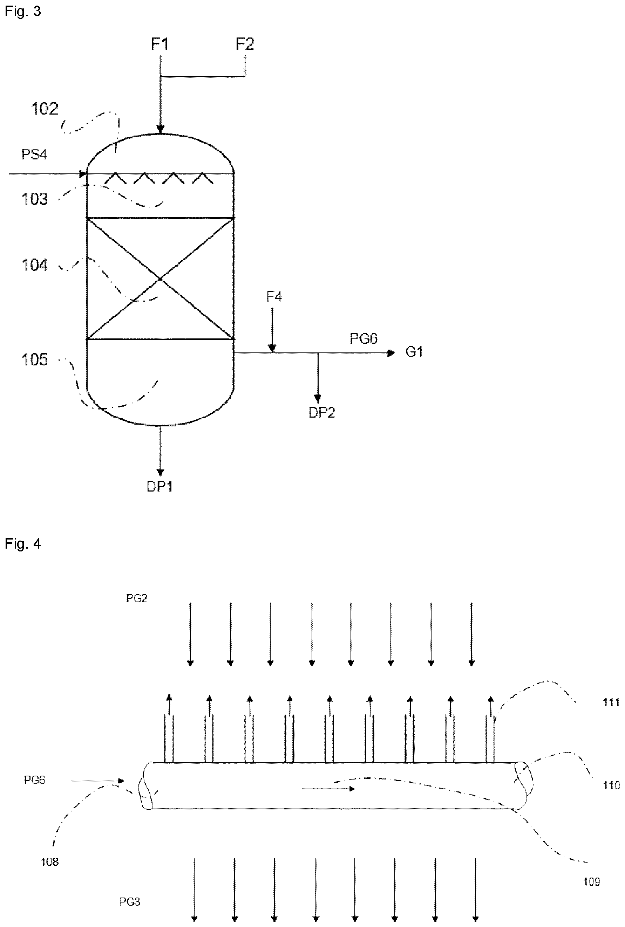

Für Schritt (e)(iv) hat es sich als vorteilhaft und damit bevorzugt erwiesen, wenn die dort erhaltene Gasmischung der in Schritt (a) resultierenden Gasmischung mittels eines Gas-Spargers derart zugeführt wird, dass die Temperatur am Austrittspunkt des Gas-Spargers unterhalb von 250 °C liegt. Der Sparger ist technisch vorteilhaft derart ausgeführt, dass das mit sauerstoffhaltigen Gasen und Wasserdampf konditionierte Gasgemisch innerhalb der Leitungen und Zuführungen einen Temperaturgradienten aufweist. Grundsätzlich wird im Bereich der Methacroleinverdampfung eine relativ niedrige Verdampfungstemperatur eingestellt. Diese sollte dabei aber hoch genug sein, um die vollständige MAL Verdampfung zu gewährleisten und eine Taupunktsunterschreitung zu vermeiden. Auf der anderen Seite sollte sie aber so niedrig sein, das keine polymeren Ablagerungen entstehen können. Je nach gewähltem Druck ist dies bei Temperaturen zwischen 50 °C und 150 °C möglich, wobei Temperaturen von kleiner 100 °C bevorzugt sind. Der Sparger kann so ausgeführt sein, dass die resultierende MAL-Mischung mit sauerstoffhaltigen Gasen und Inertgasen mit einem oder mehreren zentralen Rohrleitungen in den Reaktorenbereich geführt wird und dann eine Vielzahl von Rohren mit der Gasmischung versorgt wird. Bei industriellen Reaktoren ist es eine bevorzugte Ausführungsform, wenn mehrere Düsen und Auslassrohre mit den Spargerhauptrohren verbunden sind. In einer bevorzugten Ausführungsform sind diese Auslassrohre oder Düsen entlang dem Sparger in äquidistanten Abständen angeordnet, unterscheiden sich aber im freien Rohrdurchmesser oder Länge um einen möglichst gleichmässige Gasverteilung zu gewährleisten. Prinzipiell sehr bevorzugt ist es, dass der freie Rohrdurchmesser der am weitesten von der Hauptleitung entfernten Auslässe größer ist, als der Rohdurchmesser der Auslässe in der Nähe der Hauptleitung. Eine solche Ausführungsform ist in Fig. 4 schematisch dargestellt.

Ein wesentliches Merkmal der Vorrichtung zum Verteilung des Recycle-MAL-Gases ist eine unterschiedliche Temperatur des aus dem Sparger austretenden Gases, insbesondere dort, wo es mit dem Prozessgas der ersten Reaktorstufe vermischt wird. Hier gilt das Prinzip dass die Temperatur des Gases an weiter vom Hauptrohr entfernten Auslässen höher ist als beispielsweise an Auslässen, die näher am Hauptrohr liegen. Grundsätzlich sind Sparger und Konfiguration der Auslässe so auszulegen, dass die Gastemperatur an den äußersten Auslasspunkten eine Temperatur von 260 °C nicht überschreitet. Bevorzugt erfolgt die Auslegung so, dass die maximale Temperatur am äußersten Auslasspunkt kleiner oder gleich 240 °C ist. Naturgemäß, mit Blick auf die Anwesenheit von Spuren höher siedender Komponenten gibt es auch eine Untergrenze bezüglich der Temperaturen zu beachten, insbesondere unter Berücksichtigung der Problematik die durch Terephthalsäure ("TPA") verursacht wird. TPA sublimiert beziehungsweise desublimiert bei Temperaturen zwischen 180 °C und 220 °C, sodass bei der Auslegung des Spargers dies berücksichtigt werden muss. Werden diese Temperaturen unterschritten, kommt es zu Ablagerungen und daraus resultierend Betriebsstörungen.

Wie dem Fachmann bekannt ist, kann die Vermischung im Gegenstrom oder im Gleichstrom erfolgen, wobei grundsätzlich beide Varianten denkbar sind. Eine bevorzugte Ausführungsform ist eine Mischung im Gegenstrom der unterschiedlichen Gasströme.

Weiterhin ist es vorteilhaft und bevorzugt, aus der in Schritt (d) und (e) erhaltenen Prozessflüssigkeit die enthaltenen hochsiedenden Verunreinigungen abzutrennen und auszuschleusen.

Darüber hinaus ist es günstig, die in Schritt (b) und oder in Schritt (e) erhaltende Methacrylsäure einem Reinigungsschritt zu unterziehen. Dabei wird diese besonders bevorzugt derart durchgeführt, dass reine Methacrylsäure, die eine Reinheit grösser 99 Gew% ausweist, erhalten wird.

Die Methacrylsäure kann darüber hinaus - unabhängig von ihrer Reinheit - gereinigt und anschließend einem Veresterungsschritt, z.B. mit Methanol zu MMA, unterzogen werden.

Das erfindungsgemäße verfahren hat insbesondere folgende Vorteile gegenüber den aus dem Stand der Technik bekannten Verfahren:
1) Das Verfahren ist überraschend energiesparend, insbesondere da keine zusätzliche Energie zum Verdampfen des Recycling-Mal aufgebracht werden muss.
2) Eine Polymerisation, insbesondere des MAL kann überraschend effizient verhindert werden. Dies erfolgt insbesondere, da sich an den kritischen Stellen keine oder nur sehr kurz Flüssigkeiten, z.B. in Form von Tröpfchen oder Flüssigkeitsfilmen, bilden können.
3) Es werden überraschend keine besonderen Vorrichtungen zur Einleitung von gasförmigen oder flüssigem Strömen benötigt, da das Reaktionssystem bei relativ geringen Drücken betrieben wird.
4) Es gibt weniger kritische Stellen in der Produktionsvorrichtung, in denen potentiell kritische Mischvorgänge erfolgen. Dies ist dadurch möglich, dass dess Recycling-MAL erst mit dem Sauerstoff vermischt und danach direkt in den Sparger geleitet wird.
5) Auch in Bezug auf zwischenzeitliches Kühlen ist das erfindungsgemäße Verfahren besonders effizient, da insbesondere das Reaktionsgas der ersten Stufe im Sparger mit größeren Mengen kühleren Gases effizient und gewollt abgekühlt wird.

Neben dem beschriebenen erfindungsgemäßen Verfahren ist auch die Verwendung einer entsprechenden Produktionsvorrichtung zur Herstellung von Methacylsäure und / oder Methacrylsäureestern, Bestandteil der vorliegenden Erfindung. Eine solche Produktionsvorrichtung umfasst mindestens die folgenden Vorrichtungen:
(a) mindestens eine Versorgungsvorrichtung für mindestens eine C4-Verbindung
(b) einen ersten Reaktor, enthaltend einen ersten Oxidationskatalysator
(c) einen zweiten Reaktor, enthaltend einen zweiten Oxidationskatalysator
(d) mindestens eine Trennvorrichtung
und
(e) mindestens eine Verdampfungsvorrichtung

Darüber hinaus ist die Produktionsvorrichtung dabei durch folgende Aspekte gekennzeichnet:
(1) Die Verdampfungsvorrichtung (e) ist mit einer Packung und optional mit mindestens einem Drainage-Ventil unterhalb der Packung zur Entfernung von Flüssigkeiten ausgestattet.
(2) Die Verdampfungsvorrichtung (e) ist über mindestens eine Verteilungsvorrichtung mit mindestens einer Versorgungsvorrichtung für flüssiges Methacrolein verbunden.
(3) Die Verdampfungsvorrichtung (e) ist über einen Einlass mit mindestens einer Versorgungsvorrichtung für Luft und mindestens einer Versorgungsvorrichtung für Sauerstoff-abgereicherte Gasmischungen verbunden.
(4) Die besagte Verdampfungsvorrichtung ist über eine Gasleitung mit dem zweiten Reaktor (c) verbunden.
(5) Die Gasleitung führt über einen Einlass, umfassend einen Gas-Sparger in den zweiten Reaktor (c).
(6) Optional ist die Gasleitung darüber hinaus mit mindestens einer Versorgungseinrichtung für Wasserdampf verbunden.

Bevorzugt ist dazu eine Ausführung der Produktionsvorrichtung, in der die Trennvorrichtung (d) mindestens einen Quench-Schritt, mindestens einen Kristallisationsschritt und mindestens einen Absorptions-/Desorptionsschritt umfasst.

Unabhängig davon bevorzugt umfasst die Verteilungsvorrichtung des Aspekts (2) eine Vielzahl von Düsen.

Als besonders vorteilhaft haben sichanderen Ausführungsformen erwiesen, bei denen innerhalb der Verdampfungsvorrichtung die Einlässe für Luft und für bezüglich Sauerstoff abgereicherte Gase oberhalb der Verteilungsvorrichtung, und die Packung unterhalb der Verteilungsvorrichtung angeordnet sind.

Abschließend ist auch die Verwendung einer Produktionsvorrichtung gemäß vorangegangener Beschreibung zur Herstellung von Methacrylsäure und / oder Methacrylsäureestern, bevorzugt von MMA Bestandteil der vorliegenden Erfindung.

### Bezugszeichenliste

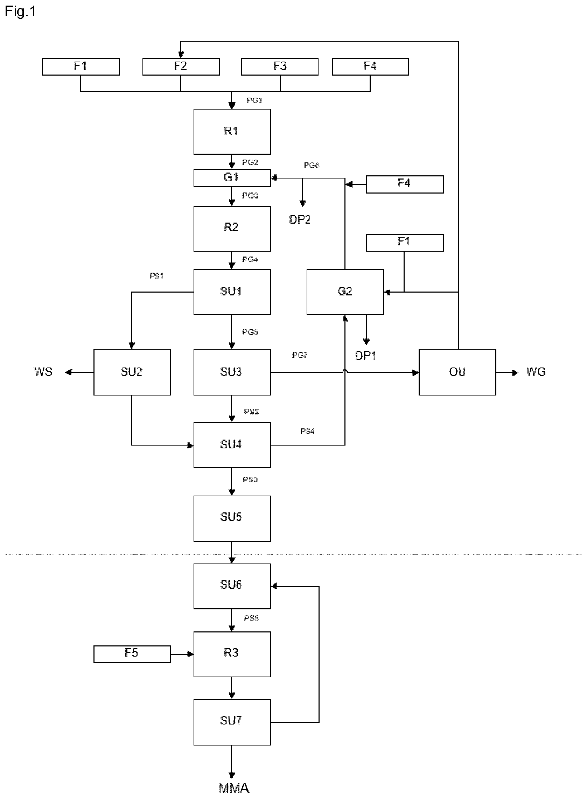

Fig.1 stellt ein schematisches Prozessflussdiagramm dar. Dabei ist die Darstellung zwar erfindungsgemäß und dient zur Verdeutlichung derselben. Die Darstellung ist aber nicht dazu geeignet, die Erfindung in irgendeiner Weise zu beschränken.
   F1: Luft
   F2: Recycle Gas
   F3: Gasstrom Isobuten und/oder tert-Butanol
   F4: Wasserdampf
   F5: Methanol
   R1: Reaktor für 1. Stufe
   R2: Reaktor für 2. Stufe
   R3: Veresterungs-Reaktor
   G1: Gas Sparger
   G2: MAL Verdampfer
   SU1: Quenchen
   SU2: Kristallisation
   SU3: Absorption
   SU4: Desorption
   SU5: Extraktion
   SU6: Reinigung Methacrylsäure
   SU7: Reinigung MMA
   OU: Offgas-Einheit
   WS: Fester Abfall
   WG: Abfallgasstrom
   DP1: Drainage 1
   DP2: Drainage 2
   PG: Prozessgas
   PS: Prozessstrom
Fig. 2 stellt beispielhaft eine spezifische Ausführungsform der Erfindung dar
   (1) Reaktor für 1. Stufe
   (2) Reaktor für 2. Stufe
   (3) Gas-Sparger
   (4) Quenchingsäule bzw. Qunechkolonne
   (5) Kristallisation
   (6) Absorptionssäule bzw. Kolonne
   (7) Desorptionssäule bzw. Kolonne
   (8) Kondensator
   (9) Methacrolein-Verdampfer
Fig. 3 stellt beispielhaft eine spezifische Ausführungsform des MAL-Verdampfers dar
   (102) Bereich mit Düsen
   (103) Freiraum zwischen Packung und Düsen
   (104) Hauptpackung
   (105) Untere Packung
Fig. 4 stellt beispielhaft eine spezifische Ausführungsform des Gas-Spargers dar
   (108) Sparger: Eintritt in Hauptleitung
   (109) Sparger: mittlere Sektion
   (110) Sparger: End-Sektion
   (111) Sparger: Austrittsrohr

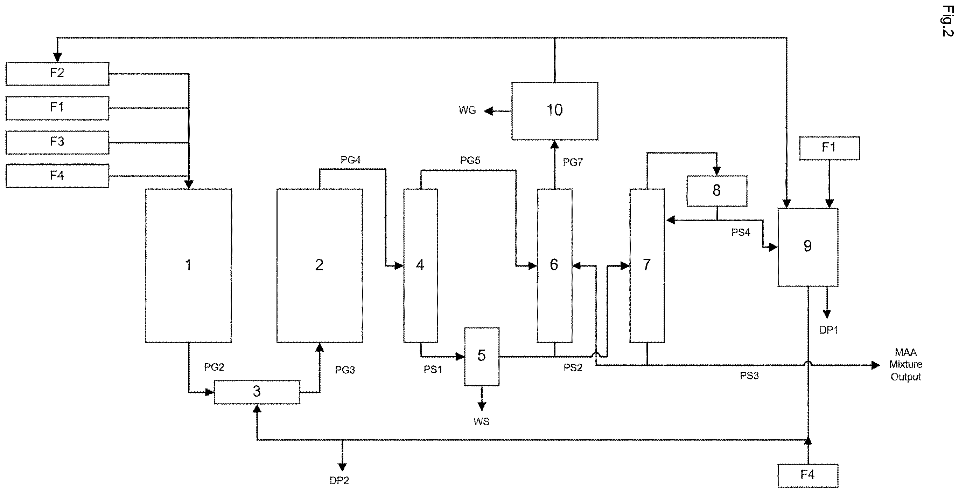

Die Bezeichnung der Prozessströme PS1 bis PS10, sowie der Prozessgase PG1 bis PG7 ergeben sich aus dem Zusammenhang der Figuren 1 bis 4, sowie aus der Beschreibung des Beispiels weiter unten.

### Beispiel

In einem Aufbau entsprechend Fig.1 wird ein Prozessgas-feed (PG1), enthaltend Isobuten (F3) als Ausgangsmaterial, Luft (F1), ein Recyclierungsgasstrom (F2), welcher Sauerstoff und inerte Gase enthält und Wasserdampf (F4) in einen Reaktor (R1) geleitet. Insgesamt weisen die Ströme dabei ein molares Verhältnis von 1:2:1.5 Isobuten zu Sauerstoff zu Wasser auf. Die Temperatur im Reaktor wurde bei 350°C und der Druck am Reaktoreinlass bei 1,2 bar Überdruck gehalten. Dabei ergab sich eine Raumzeitgeschwindigkeit ("Gaseous hourly Space velocity", GHSV) von 1,000 h⁻¹. Die Reaktion wurde in einem Rohrbündel Reaktor mit einem auf Molybdänoxid basierenden Katalysator, der gemäß US 2007/0010394 hergestellt wurde, durchgeführt. Das resultierende Prozessgas (PG2) wurde mit einer Temperatur von 343 °C erhalten. Dieses Prozessgas hatte folgende Zusammensetzung: 4,8 Vol% Methacrolein, 0,74 mol% CO, 0,21 mol% Methacrylsäure, 0,21 mol% Essigsäure, 0,12 mol% Aceton, 0,21 mol% Acetaldehyd, 0,04 mol% Acrolein, 0,03 mol% Formaldehyd, 0,03 mol% Acrylsäure, 250 ppm Isobuten und 3,5 Vol% Sauerstoff. Die Isobuten Umsatzrate betrug 99,6% und die Ausbeute an Methacrolein betrug 79,6%.

Im nächsten Schritt wird das Prozessgas (PG2) mit dem zusätzlichen Prozessgas (PG6) mit einem Volumenflussverhältnis (PG6) zu (PG2) von 1 zu 0,8 in Kontakt gebracht und gemischt. Das Mischen erfolgt durch einen Gas Sparger (G1). Bei dem zusätzlichen Prozessgas (PG6) handelt es sich um teilweise recyceltes Methacrolein, das nach Verdampfung und Vermischung mit weiteren im geführten Recycle Gas, Luft und Wasserdampf. eine Temperatur von 80 °C aufweist. Das Prozessgas (PG6) hat folgende Zusammensetzung: 1,41 mol% organischer Bestandteile, beinhaltend 1,2 mol% Methacrolein, 0,03 mol% Methacrylsäure, 0,09 mol% Aceton, 0,05 mol% Acetaldehyd, 0,04 mol% Acrolein, sowie 17 mol% Sauerstoff und 13 mol% Wasser.

Das bei diesem Mischen resultierende Prozessgas (PG3) hat nach Mischung und Inkontaktbringen mit Reaktorgas (PG2) eine Temperatur von 245 °C und ein molares Verhältnis von Methacrolein zu Sauerstoff zu Wasser von 1 zu 2,5 zu 4,1. In Summe enthält das Prozessgas (PG3) 4,48 mol% organischer Bestandteile, welches ich überwiegend wie folgt zusammensetzen: 3,44 mol% Methacrolein, 0,41 mol% CO, 0,12 mol% Methacrylsäure, 0,12 mol% Essigsäure, 0,15 mol% Aceton, 0,15 mol% Acetaldehyd, 0,06 mol% Acrolein, 0,02 mol% Formaldehyd und 0,01 mol% Acrylsäure. Dieses Prozessgas (PG3) wurde nun durch einen zweiten Reaktor (R2), geleitet. Der Reaktor (R2) war teilweise mit einem Katalysator, bei dem es sich um eine Mischung beinhaltend Phosphormolybdat handelt, gefüllt. Der Katalysator wurde gemäß US 2007/0010394 hergestellt und die Reaktion erfolgte bei 300°C (Salztemperatur) und einer Durchflussrate von 1,000 h⁻¹. Der Umsatz des Methacroleins in Reaktor R2 betrug ungefähr 80%, die Selektivität zu Methacrylsäure betrug im Betriebszeitraum durchschnittlich 84% (Schwankungsbreite der unterschiedlichen Betriebsmessungen und Gasanalysen +/- 1%). Ein bei der Reaktion gebildetes Reaktionsgas (PG4) hat folgende Zusammensetzung: 0,7 Vol% Methacrolein, 1 mol% CO, 2,28 mol% Methacrylsäure, 0,31 mol% Essigsäure, 0,12 mol% Aceton, 0,21 mol% Acetaldehyd, 0,04 mol% Acrolein, 0,03 mol% Formaldehyd, 0,03 mol% Acrylsäure und 6,5 vol% Sauerstoff. Die Gastemperatur nach Austritt des Methacrylsäure und MAL haltigen Prozessgases (PG4) beträgt etwa 302°C und wird mittels eines Gaskühlers auf 230°C abgekühlt, bevor es in der Quenchkolonne auf 80°C abgekühlt wird. Im Sumpf der Quenchkolonne wird hauptsächlich Methacrylsäure, Essigsäure, TPA und Acrylsäure und andere relativ zum Methacrolein höher siedende Komponenten absorbiert bzw. kondensiert zusammen mit einem Teil des Prozesswassers.

Das Methacrolein haltige Reaktionsgas wird anschließend durch eine Quenchkolonne (SU1) geleitet und auf eine Temperatur von kleiner 30°C abgekühlt. In der Quenchkolonne (SU1) wird ein Gasgemisch (PG5) gebildet und weiter mit mehreren externen Kühlern auf 13 °C abgekühlt. Der größte Teil der Methacrylsäure und des Wassers werden in der Quenchkolonne kondensiert. Die dabei gebildete Quenchflüssigkeit (QL) wird am Kolonnenboden (PS1) gesammelt und enthält 35 Gew% Methacrylsäure, 0,2% Methacrolein, 1000 ppm TPA (Terephthalsäure) und Wasser. Die Flüssigkeit wird auf 13 °C abgekühlt, bevor sie in eine TPA Kristallisation (SU2) geleitet wird. In dieser Kristallisation (SU2) wird die übersättigte TPA auskristallisiert und die Flüssigkeit mit einer TPA Filtrationseinheit filtriert. TPA wird darauf als Abfallfeststoff (WS) entnommen. Das Filtrat wiederum wird in die Methacrolein Recovery Kolonne (SU4) geleitet, jetzt weitgehend frei von TPA.

Das Gasgemisch (PG5) aus der Quenchkolonne wird darauf in den Sumpf der Methacrolein Absorptionskolonne (SU3) geleitet. In dieser Absorptionskolonne (SU3) wird der Prozesstrom (PS3) als Absorbermedium des Methacroleins eingesetzt. Der Prozessstrom (PS3) wird vor der Einleitung in den Kolonnenkopf auf 17°C abgekühlt. Der größte Teil des Methacroleins in der Gasphase (PG5) wird dabei in den Prozessstrom (PS2), der im Gegenstrom betrieben wird, absorbiert. Das über Kopf entnommene austretende Gasgemisch, welches den größten Teil der nicht absorbierten bzw. kondensierten Gasphase der MAL Absorptionskolonne (PG7) aufweist, wird gewaschen und darauf in mehreren Demistern und über die Wärme der Verbrennungsanlage auf 300°C aufgeheizt. Letztendlich wird es in einer Verbrennungsanlage (OU, Offgas Unit), bestückt mit einem Standard-Pt-Pd Verbrennungskatalysator geleitet und dort alle organischen Bestandteile verbrannt. Die Verbrennungsanlage wird regenerativ betrieben, das heisst die Abwärme der Reaktion wird genutzt zur Vorwärmung der Eintrittsgase. Ein Teil des austretenden Abgases der Verbrennungsanlage wird in einem Abgaskompressor verdichtet und in das Oxidationsreaktionssystem recycliert. Diese Gasphase wird im Weiteren als Rezyklier-Gas (F2) bezeichnet. Der verbleibende Teil wird als Abgas (WG, Waste Gas) in die Atmosphäre entlassen. Der Prozessstrom (PS2) aus dem Sumpf der Methacroleinabsorptionskolonne (SU3) sowie die Flüssigphase der TPA-Filtration werden jeweils in die MAL-Recovery-Kolonne (SU4) geleitet, um dort nicht umgesetztes Methacrolein zurückzugewinnen. Diese Kolonne wird unter einem Unterdruck von 350 mbar absolut betrieben. Die Temperatur der Flüssigkeit im Sumpf liegt dabei bei ca. 80°C und die Temperatur der Gasphase am Kopf der Kolonne liegt bei ca. 40 °C, dies entspricht der Brüden Temperatur vor dem Kondensator. Ein Teil der Flüssigkeit aus dem Sumpf (PS3) wird als Absorber Medium wiederverwendet. Der Rest des Sumpfes (PS3), welcher 33 Gew% Methacrylsäure, 80 ppm Methacrolein und 250 ppm TPA enthält, wird in die Methacrylsäure Aufarbeitungsanlage weitergeleitet. Der größte Teil des Methacroleins in der Gasphase wird über den Kopf der Kolonne abdistilliert, auf 30 °C gekühlt, und als Methacrolein-haltige Flüssigkeit erhalten. Ein Teil der Flüssigkeit aus dem Kondensator wird zurück in die Kolonne als Rücklauf geleitet. Der andere Teil der Flüssigphase (PS4) wird in den Methacroleinverdampfer als Recyclingstrom geleitet.

Der Methacrolein-Recyclingstrom (PS4) enthält 85 Gew% Methacrolein, 5 Gew% Aceton, 3 Gew% Acrolein und 0,5 Gew% Methacrylsäure, sowie Inhibitoren. Bevor er in den Methacroleinverdampfer geleitet wird, wird der Recyclingstrom für die Zwischenlagerung auf 3 °C abgekühlt. Der Methacrolein-Recyclingstrom (PS4) wird anschließend in einen mit einem Zersteubersystem ausgestatteten Flüssigkeitsverteiler gepumpt. Hier werden Tröpfchen generiert, die auf die Packung im Methacroleinverdampfer gesprüht werden. Dadurch wird eine homogene, gleichmäßige Oberflächenverteilung für eine schnelle, vollständige Verdampfung realisiert. Die zugeleitete Luft (F1) und das rezyklierte Gas (F2) werden nach einer Komprimierung auf 90 °C abgekühlt. Die resultierende Zusammensetzung der Gasphase nach Mischung von F2 und F1 enthält daraufhin 17 vol% Sauerstoff. Die komprimierte Gasmischung wird dann in den Kopf des Methacroleinverdampfers geleitet. Das Verdampfen erfolgt bei einem Druck von 1,2 bar Überdruck und man erhält ein resultierendes sauerstoffhaltiges Gas mit einer Temperatur von 80 °C, die oberhalb des Taupunktes von MAL beim gewählten Druck liegt. Bei der Verdampfung ist es wichtig, dass die Verdampfung dicht an oder oberhalb des Taupunktes der organischen Mischung erfolgt. Damit wird eine benötigte vollständige Verdampfung möglichst aller organischer Bestandteile gesichert. Bei dem Verdampfungsvorgang ist es möglich, dass ein Teil der organischen Bestandteile mit einem gegenüber zum Methacrolein höherem Siedepunkt - wie z.B. die Inhibitoren, Methacrylsäure oder Terephthalsäure - nicht vollständig beziehungsweise nur in Spuren in die Gasphase übergehen. Daher wird der Methacroleinverdampfer regelmäßig mit Wasser gesäubert und dieses aus einem Drainagepunkt (DP1) vom Sumpf des Methacroleinverdampfers und von einem späteren zweiten Drainagepunkt (DP2) entnommen. Die so gesäuberte Methacrolein-Phase wird schließlich in die Gasverteilungsvorrichtung, dem Sparger (G1) geleitet. Methacrolein, Acrolein, Aceton und andere niedrigsiedende Komponenten werden verdampft und als Gasphase mit hoher Geschwindigkeit in den Sparger (G1) geleitet, optional mit zusätzlichem Wasserdampf (F4). Der Sparger leitet und verteilt die Gasmischung direkt unterhalb die Ausgangszone des ersten Reaktors (R1). Bei dem direkten Kontakt der Gasverteilungsvorrichtung sowie der hiermit verbundenen Rohrleitungsmimik mit dem heißen Reaktionsgas (PG2) der ersten Reaktionszone wird der gasförmige Rezyklier-Methacrolein Stroms (PG6), beide Ströme vereinigen sich zum resltierenden Gasstrom (PG3) auf eine Temperatur von 238 °C vorgeheizt. Diese Temperaturmessung bezieht sich auf die MischTemperatur nahe am äußeren Auslass Röhrchens.

## Patentansprüche

1. Verfahren zur Herstellung von Methacrylsäure und / oder Methacrylsäureestern, umfassend die Verfahrensschritte
(a) Umsetzung von Isobuten, tert-Butanol oder einer Mischung enthaltend Isobuten und/oder tert-Butanol in einem ersten katalytischen Gasphasenoxidationsschritt zu einem hauptsächlich Methacrolein enthaltenden Reaktionsprodukt,
(b) Umsetzung des hauptsächlich Methacrolein enthaltenden Reaktionsprodukts aus Verfahrensschritt (a) in einem zweiten katalytischen Gasphasenoxidationsschritt zu einem überwiegend aus Methacrylsäure und nicht umgesetztem Methacrolein bestehenden gasförmigen Reaktionsprodukt,
(c) Abtrennung, Vorreinigung und Kondensation eines Teils des in Verfahrensschritt (b) im gasförmigen Reaktionsprodukt anfallenden nicht umgesetzten Methacroleins zu einer flüssigen Recycle-Methacrolein-Fraktion,
(d) Überführung der Recycle-Methacrolein-Fraktion in einen gasförmigen Strom und
(e) Umsetzung dieses gasförmigen Recycle-Methacroleinstroms in einem katalytischen Oxidationsschritt,
**dadurch gekennzeichnet, dass** Verfahrensschritt (d) die Verfahrensaspekte
i) gleichmäßige Verteilung der in Schritt c) erhaltenen flüssigen Recycle-Methacrolein-Fraktion in Form von Tröpfchen auf der Oberfläche einer Packung zum Erhalt eines Flüssigkeitsfilms,
ii) Inkontaktbringen des in Schritt (i) erhaltenen Flüssigkeitsfilms mit einem 3 bis 21 Vol% Sauerstoff enthaltenden Gasstrom, der eine Temperatur aufweist, die 10 bis 150 °C höher ist als die Temperatur des flüssigen Methacroleins, unter Erhalt einer Recycle-Methacrolein enthaltenden Gasmischung,
iii) optional Vermischung des gemäß (ii) erhaltenen Gasstroms mit zusätzlichem Wasserdampf und
iv) Mischen des resultierende Gasstroms, enthaltend Methacrolein, Sauerstoff, Wasserdampf, Stickstoff, sowie andere organische Verbindungen mit 1 bis 6 Kohlenstoffatomen mit dem Reaktionsprodukt der ersten Oxidationstufe (a)
umfasst.

2. Verfahren gemäß Anspruch 1, **dadurch gekennzeichnet, dass** es sich bei den in den Schritten (a) und (b) beschriebenen Oxidationsschritten um eine in Gegenwart einer sauerstoffhaltigen Gasmischung und Wasserdampf in einem Rohrreaktor über einem heterogenen Kontakt durchgeführte katalytische Gasphasenoxidation handelt.

3. Verfahren gemäß Anspruch 1 oder 2, **dadurch gekennzeichnet, dass** in Schritt (c) mindestens 80 % der gebildeten Methacrylsäure vom nicht umgesetzten Methacrolein abgetrennt werden.

4. Verfahren gemäß mindestens einem der Ansprüche 1 bis 3, **dadurch gekennzeichnet, dass** die Abtrennung und Vorreinigung gemäß Schritt (c) realisiert ist durch
(i) Quenchen der Reaktionsmischung unter Erhalt eines Prozessgases und einer Prozessflüssigkeit,
(ii) Abtrennen mindestens eines Teils der Nebenprodukte aus der Prozessflüssigkeit durch Kristallisation und
(iii) sequentielle Durchführung von Absorptions- und Desorptionsschritten unter Erhalt einer flüssigen Recycle-Methacrolein-Fraktion und einer Stickstoff, Sauerstoff und Wasserdampf enthaltenden Recycle-Gas-Phase.

5. Verfahren gemäß mindestens einem der Ansprüche 1 bis 4, **dadurch gekennzeichnet, dass** Verfahrensschritt (d) die Recycle-Methacrolein-Fraktion mittels ein oder mehreren Düsen in Gegenwart von ein oder mehreren Gasströmen mit unterschiedlichen Sauerstoffgehalten versprüht und verteilt wird, wobei die entstehenden Tröpfchen einen durchschnittlichen Durchmesser zwischen 100 und 1000 µm aufweisen und ein Gasstrom einen Sauerstoffgehalt kleiner 10 Vol% und optional ein anderer Gasstrom einen Sauerstoffgehalt größer 10 Vol% aufweist.

6. Verfahren gemäß mindestens einem der Ansprüche 1 bis 5, **dadurch gekennzeichnet, dass** die in Schritt (c) erhaltene flüssige Recycle-Methacrolein-Fraktion 65 bis 99 Gew% Methacrolein, 0 bis 5 Gew% Methacrylsäure, 0 bis 5 Gew% Wasser, bis 5000 ppm Stabilisatoren, Terephthalsäure, nicht flüchtige Verbindungen, 0,5 bis 10 Gew% Aceton und in Summe 0 bis 5 Gew% Essigsäure, Acetaldehyd und Acrolein enthält.

7. Verfahren gemäß mindestens einem der Ansprüche 1 bis 6, **dadurch gekennzeichnet, dass** in Schritt (e)(i) mindestens 50 % der Recycle-Methacrolein-Tröpfchen, mit Tröpfchengrößen kleiner als 500 µm, auf der Packung verteilt werden.

8. Verfahren gemäß mindestens einem der Ansprüche 1 bis 7, **dadurch gekennzeichnet, dass** die in der in Schritt (d) und (e) erhaltenen Prozessflüssigkeit enthaltenen hochsiedenden Verunreinigungen abgetrennt und ausgeschleust werden.

9. Verfahren gemäß mindestens einem der Ansprüche 1 bis 8, **dadurch gekennzeichnet, dass** der in Schritt (e)(ii) verwendete 3 bis 21 Vol% Sauerstoff enthaltende Gasstrom durch Vermischung von in Schritt (a) und / oder Schritt (b) erhaltenem rückgeführtem Prozessgas und zusätzlicher Luft erhalten wird.

10. Verfahren gemäß mindestens einem der Ansprüche 1 bis 9, **dadurch gekennzeichnet, dass** die in Schritt (e)(iv) erhaltene Gasmischung der in Schritt (a) resultierenden Gasmischung mittels eines Gas-Spargers derart zugeführt wird, dass die Temperatur am Austrittspunkt des Gas-Spargers unterhalb von 250 °C liegt.

11. Verfahren gemäß mindestens einem der Ansprüche 1 bis 10, **dadurch gekennzeichnet, dass** die in Schritt (b) und oder in Schritt (e) erhaltende Methacrylsäure einem Reinigungsschritt unterzogen wird, wobei reine Methacrylsäure, die eine Reinheit grösser 99 Gew% aufweist, erhalten wird.

12. Verfahren gemäß mindestens einem der Ansprüche 1 bis 11, **dadurch gekennzeichnet, dass** die Methacrylsäure gereinigt und anschließend einem Veresterungsschritt unterzogen wird.

13. Verwendung einer Produktionsvorrichtung zur Herstellung von Methacylsäure und / oder Methacrylsäureestern, umfassend
(a) mindestens eine Versorgungsvorrichtung für mindestens eine C4-Verbindung,
(b) einen ersten Reaktor, enthaltend einen ersten Oxidationskatalysator,
(c) einen zweiten Reaktor, enthaltend einen zweiten Oxidationskatalysator,
(d) mindestens eine Trennvorrichtung und
(e) mindestens eine Verdampfungsvorrichtung,
**dadurch gekennzeichnet, dass**
(1) die Verdampfungsvorrichtung (e) mit einer Packung und optional mit mindestens einem Drainage-Ventil unterhalb der Packung zur Entfernung von Flüssigkeiten ausgestattet ist,
(2) die Verdampfungsvorrichtung (e) über mindestens eine Verteilungsvorrichtung mit mindestens einer Versorgungsvorrichtung für flüssiges Methacrolein verbunden ist,
(3) die Verdampfungsvorrichtung (e) über einen Einlass mit mindestens einer Versorgungsvorrichtung für Luft und mindestens einer Versorgungsvorrichtung für Sauerstoff-abgereicherte Gasmischungen verbunden ist,
(4) die besagte Verdampfungsvorrichtung über eine Gasleitung mit dem zweiten Reaktor (c) verbunden ist,
(5) die Gasleitung über einen Einlass umfassend einen Gas-Sparger in den zweiten Reaktor (c) führt und
(6) optional die Gasleitung darüber hinaus mit mindestens einer Versorgungseinrichtung für Wasserdampf verbunden ist.

14. Verwendung einer Produktionsvorrichtung gemäß Anspruch 13, **dadurch gekennzeichnet, dass** die Trennvorrichtung (d) mindestens einen Quench-Schritt, mindestens einen Kristallisationsschritt und mindestens einen Absorptions-/Desorptionsschritt umfasst.

15. Verwendung einer Produktionsvorrichtung gemäß Anspruch 13 oder 14, **dadurch gekennzeichnet, dass** die Verteilungsvorrichtung einer Vielzahl von Düsen umfasst.

16. Verwendung einer Produktionsvorrichtung gemäß der mindestens einem der Ansprüche 13 bis 15, **dadurch gekennzeichnet, dass** innerhalb der Verdampfungsvorrichtung die Einlässe für Luft und für bezüglich Sauerstoff abgereicherte Gase oberhalb der Verteilungsvorrichtung, und die Packung unterhalb der Verteilungsvorrichtung angeordnet sind.

## Claims

1. Process for preparing methacrylic acid and/or methacrylic esters, comprising the process steps
(a) conversion of isobutene, tert-butanol or a mixture containing isobutene and/or tert-butanol in a first catalytic gas-phase oxidation step to give a reaction product mainly containing methacrolein,
(b) conversion of the reaction product mainly containing methacrolein from process step (a) in a second catalytic gas-phase oxidation step to give a gaseous reaction product consisting predominantly of methacrylic acid and unconverted methacrolein,
(c) removal, preliminary purification and condensation of a portion of the unconverted methacrolein accumulating in the gaseous reaction product in process step (b) to form a liquid recycle methacrolein fraction,
(d) transfer of the recycle methacrolein fraction into a gaseous stream and
(e) conversion of this gaseous recycle methacrolein stream in a catalytic oxidation step,
**characterized in that** process step (d) comprises the process aspects of
i) uniform distribution of the liquid recycle methacrolein fraction obtained in step c) in the form of droplets on the surface of a packing to obtain a liquid film,
ii) contacting of the liquid film obtained in step (i) with a gas stream containing 3% to 21% by volume of oxygen and having a temperature which is 10 to 150°C higher than the temperature of the liquid methacrolein, to obtain a gas mixture containing recycle methacrolein,
iii) optional mixing of the gas stream obtained according to (ii) with additional steam and
iv) mixing of the resulting gas stream, containing methacrolein, oxygen, steam, nitrogen and other organic compounds having 1 to 6 carbon atoms, with the reaction product of the first oxidation stage (a).

2. Process according to Claim 1, **characterized in that** the oxidation steps described in steps (a) and (b) involve a catalytic gas-phase oxidation conducted over a heterogeneous catalyst in the presence of an oxygen-containing gas mixture and steam in a tubular reactor.

3. Process according to Claim 1 or 2, **characterized in that** in step (c) at least 80% of the methacrylic acid formed is removed from the unconverted methacrolein.

4. Process according to at least one of Claims 1 to 3, **characterized in that** the removal and preliminary purification according to step (c) is implemented by
(i) quenching the reaction mixture to obtain a process gas and a process liquid,
(ii) removing at least a portion of the byproducts from the process liquid by crystallization and
(iii) sequentially conducting absorption and desorption steps to obtain a liquid recycle methacrolein fraction and a nitrogen-, oxygen- and steam-containing recycle gas phase.

5. Process according to at least one of Claims 1 to 4, **characterized in that** in process step (d) the recycle methacrolein fraction is sprayed and distributed by means of one or more nozzles in the presence of one or more gas streams with differing oxygen contents, wherein the droplets formed have an average diameter of between 100 and 1000 µm and one gas stream has an oxygen content of less than 10% by volume and optionally another gas stream has an oxygen content of greater than 10% by volume.

6. Process according to at least one of Claims 1 to 5, **characterized in that** the liquid recycle methacrolein fraction obtained in step (c) contains 65% to 99% by weight of methacrolein, 0% to 5% by weight of methacrylic acid, 0% to 5% by weight of water, up to 5000 ppm of stabilizers, terephthalic acid, nonvolatile compounds, 0.5% to 10% by weight of acetone and a total of 0% to 5% by weight of acetic acid, acetaldehyde and acrolein.

7. Process according to at least one of Claims 1 to 6, **characterized in that**, in step (e) (i), at least 50% of the recycle methacrolein droplets, having droplet sizes of less than 500 µm, are distributed on the packing.

8. Process according to at least one of Claims 1 to 7, **characterized in that** the high-boiling contaminants present in the process liquid obtained in step (d) and (e) are removed and discharged.

9. Process according to at least one of Claims 1 to 8, **characterized in that** the gas stream containing 3% to 21% by volume of oxygen which is used in step (e) (ii) is obtained by mixing returned process gas obtained in step (a) and/or step (b) and additional air.

10. Process according to at least one of Claims 1 to 9, **characterized in that** the gas mixture obtained in step (e) (iv) is introduced to the gas mixture resulting in step (a) by means of a gas sparger in such a way that the temperature at the exit point of the gas sparger is below 250°C.

11. Process according to at least one of Claims 1 to 10, **characterized in that** the methacrylic acid obtained in step (b) and/or in step (e) is subjected to a purification step to obtain pure methacrylic acid having a purity of greater than 99% by weight.

12. Process according to at least one of Claims 1 to 11, **characterized in that** the methacrylic acid is purified and then subjected to an esterification step.

13. Use of a production apparatus for the preparation of methacrylic acid and/or methacrylic esters, comprising
(a) at least one supply apparatus for at least one C4 compound,
(b) a first reactor, containing a first oxidation catalyst,
(c) a second reactor, containing a second oxidation catalyst,
(d) at least one separation apparatus and
(e) at least one evaporation apparatus,
**characterized in that**
(1) the evaporation apparatus (e) is equipped with a packing and optionally with at least one drainage valve below the packing for the removal of liquids,
(2) the evaporation apparatus (e) is connected to at least one supply apparatus for liquid methacrolein via at least one distribution apparatus,
(3) the evaporation apparatus (e) is connected via an inlet to at least one supply apparatus for air and at least one supply apparatus for oxygen-depleted gas mixtures,
(4) said evaporation apparatus is connected to the second reactor (c) via a gas conduit,
(5) the gas conduit leads into the second reactor (c) via an inlet comprising a gas sparger and
(6) optionally, the gas conduit is additionally connected to at least one supply device for steam.

14. Use of a production apparatus according to Claim 13, **characterized in that** the separation apparatus (d) comprises at least one quenching step, at least one crystallization step and at least one absorption/desorption step.

15. Use of a production apparatus according to Claim 13 or 14, **characterized in that** the distribution apparatus comprises a plurality of nozzles.

16. Use of a production apparatus according to at least one of Claims 13 to 15, **characterized in that**, inside the evaporation apparatus, the inlets for air and for gases depleted in oxygen are arranged above the distribution apparatus, and the packing is arranged below the distribution apparatus.

## Revendications

1. Procédé pour la préparation d'acide méthacrylique et/ou d'esters d'acide méthacrylique, comprenant les étapes de procédé
(a) transformation d'isobutène, de tert-butanol ou d'un mélange contenant de l'isobutène et/ou du tert-butanol dans une première étape d'oxydation catalytique en phase gazeuse pour donner un produit de réaction contenant principalement de la méthacroléine,
(b) transformation du produit de réaction contenant principalement de la méthacroléine de l'étape de procédé (a) dans une deuxième étape d'oxydation catalytique en phase gazeuse pour donner un produit de réaction gazeux constitué majoritairement d'acide méthacrylique et de méthacroléine non transformée,
(c) séparation, pré-purification et condensation d'une partie de la méthacroléine non transformée produite dans le produit de réaction gazeux dans l'étape de procédé (b) pour donner une fraction liquide de méthacroléine recyclée,
(d) conversion de la fraction de méthacroléine recyclée en un flux gazeux et
(e) transformation de ce flux gazeux de méthacroléine recyclée dans une étape d'oxydation catalytique,
**caractérisé en ce que** l'étape de procédé (d) comprend les aspects de procédé suivant :
i) distribution uniforme de la fraction liquide de méthacroléine recyclée obtenue dans l'étape c) sous forme de gouttelettes sur la surface d'un garnissage pour obtenir un film de liquide,
ii) mise en contact du film de liquide obtenu dans l'étape (i) avec un flux de gaz contenant 3 à 21 % en volume d'oxygène, qui présente une température qui est 10 à 150 °C plus élevée que la température de la méthacroléine liquide, avec obtention d'un mélange de gaz contenant de la méthacroléine recyclée,
iii) éventuellement mélange du flux de gaz obtenu selon (ii) avec de la vapeur d'eau supplémentaire et
iv) mélange du flux de gaz résultant, contenant de la méthacroléine, de l'oxygène, de la vapeur d'eau, de l'azote, ainsi que d'autres composés organiques comportant 1 à 6 atomes de carbone avec le produit de réaction de la première étape d'oxydation (a).

2. Procédé selon la revendication 1, **caractérisé en ce que** les étapes d'oxydation décrites dans les étapes (a) et (b) sont une oxydation en phase gazeuse catalytique mise en œuvre par le biais d'un contact hétérogène dans un réacteur tubulaire en présence de vapeur d'eau et d'un mélange de gaz contenant de l'oxygène.

3. Procédé selon la revendication 1 ou 2, **caractérisé en ce que** dans l'étape (c) au moins 80 % de l'acide méthacrylique formé sont séparés de la méthacroléine qui n'a pas réagi.

4. Procédé selon au moins l'une des revendications 1 à 3, **caractérisé en ce que** la séparation et la pré-purification selon l'étape (c) sont réalisées par
(i) trempe du mélange réactionnel avec obtention d'un gaz de processus et d'un liquide de processus,
(ii) séparation d'au moins une partie des produits secondaires du liquide de processus par cristallisation et
(iii) réalisation séquentielle d'étapes d'absorption et de désorption avec obtention d'une fraction liquide de méthacroléine recyclée et d'une phase gazeuse recyclée contenant de l'azote, de l'oxygène et de la vapeur d'eau.

5. Procédé selon au moins l'une des revendications 1 à 4, **caractérisé en ce que** dans l'étape de procédé (d) la fraction de méthacroléine recyclée est pulvérisée et répartie au moyen d'une ou plusieurs buses en présence d'un ou plusieurs flux de gaz dotés de teneurs en oxygène différentes, les gouttelettes formées présentant un diamètre moyen compris entre 100 et 1 000 µm et un flux de gaz présentant une teneur en oxygène inférieure à 10 % en volume et éventuellement un autre flux de gaz présentant une teneur en oxygène supérieure à 10 % en volume.

6. Procédé selon au moins l'une des revendications 1 à 5, **caractérisé en ce que** la fraction liquide de méthacroléine recyclée obtenue dans l'étape (c) contient 65 à 99 % en poids de méthacroléine, 0 à 5 % en poids d'acide méthacrylique, 0 à 5 % en poids d'eau, jusqu'à 5 000 ppm de stabilisants, d'acide téréphtalique, de composés non volatils, 0,5 à 10 % en poids d'acétone et au total 0 à 5 % en poids d'acide acétique, d'acétaldéhyde et d'acroléine.

7. Procédé selon au moins l'une des revendications 1 à 6, **caractérisé en ce que** dans l'étape (e)(i) au moins 50 % des gouttelettes de méthacroléine recyclée, comportant des tailles de gouttelettes inférieures à 500 µm, sont réparties sur le garnissage.

8. Procédé selon au moins l'une des revendications 1 à 7, **caractérisé en ce que** les impuretés à point d'ébullition élevé contenues dans le liquide de processus obtenu dans l'étape (d) et (e) sont séparées et évacuées.

9. Procédé selon au moins l'une des revendications 1 à 8, **caractérisé en ce que** le flux de gaz contenant 3 à 21 % en volume d'oxygène utilisé dans l'étape (e)(ii) est obtenu par mélange de gaz de processus recyclé obtenu dans l'étape (a) et/ou l'étape (b) et d'air supplémentaire.

10. Procédé selon au moins l'une des revendications 1 à 9, **caractérisé en ce que** le mélange de gaz obtenu dans l'étape (e)(iv) est alimenté au mélange de gaz résultant de l'étape (a) au moyen d'un aspergeur de gaz de telle manière que la température au niveau du point de sortie de l'aspergeur de gaz se situe en dessous de 250 °C.

11. Procédé selon au moins l'une des revendications 1 à 10, **caractérisé en ce que** l'acide méthacrylique obtenu dans l'étape (b) et/ou dans l'étape (e) est soumis à une étape de purification, de l'acide méthacrylique pur étant obtenu qui présente une pureté supérieure à 99 % en poids.

12. Procédé selon au moins l'une des revendications 1 à 11, **caractérisé en ce que** l'acide méthacrylique est purifié et ensuite soumis à une étape d'estérification.

13. Utilisation d'un dispositif de production de préparation d'acide méthacrylique et/ou d'esters d'acide méthacrylique, comprenant
(a) au moins un dispositif d'alimentation pour au moins un composé en C4,
(b) un premier réacteur, contenant un premier catalyseur d'oxydation,
(c) un deuxième réacteur, contenant un deuxième catalyseur d'oxydation,
(d) au moins un dispositif de séparation et
(e) au moins un dispositif d'évaporation,
**caractérisée en ce que**
(1) le dispositif d'évaporation (e) est équipé d'un garnissage et éventuellement d'au moins une soupape de drainage en dessous du garnissage pour l'élimination de liquides,
(2) le dispositif d'évaporation (e) est relié par le biais d'au moins un dispositif de répartition à au moins un dispositif d'alimentation de méthacroléine liquide,
(3) le dispositif d'évaporation (e) est relié par le biais d'un orifice d'entrée à au moins un dispositif d'alimentation d'air et au moins un dispositif d'alimentation en mélanges de gaz enrichis en oxygène,
(4) ledit dispositif d'évaporation est relié par le biais d'une conduite de gaz à un deuxième réacteur (c),
(5) la conduite de gaz conduit dans le deuxième réacteur (c) par le biais d'un orifice d'entrée comprenant un aspergeur de gaz,
(6) éventuellement la conduite de gaz est en outre reliée à au moins un dispositif d'alimentation en vapeur d'eau.

14. Utilisation d'un dispositif de production selon la revendication 13, **caractérisée en ce que** le dispositif de séparation (d) comprend au moins une étape de trempe, au moins une étape de cristallisation et au moins une étape d'absorption/désorption.

15. Utilisation d'un dispositif de production selon la revendication 13 ou 14, **caractérisée en ce que** le dispositif de répartition comprend une pluralité de buses.

16. Utilisation d'un dispositif de production selon au moins l'une des revendications 13 à 15, **caractérisée en ce qu'**à l'intérieur du dispositif d'évaporation, les orifices d'entrée pour l'air et pour des gaz enrichis en oxygène sont agencés au-dessus du dispositif de répartition, et le garnissage est agencé en dessous du dispositif de répartition.
